# EUROPÄISCHE PATENTANMELDUNG

(11) **EP 1 180 685 A1**
(43) Veröffentlichungstag der Anmeldung: **20.02.2002**
(21) Anmeldenummer: 00250268.0
(22) Anmeldetag: 09.08.2000
(51) Int. Cl.: G01N 33/28, G01N 7/18

(54) **Gerät zur quantitativen Bestimmung eines Wassergehaltes und einer Basenzahl**

(71) Anmelder: Martechnic GmbH, 22525 Hamburg (DE)
(72) Erfinder: Winkler, Mattias, 25337 Elmshorn (DE)
(74) Vertreter: Patentanwälte Wenzel & Kalkoff

(57) **Zusammenfassung**

Ein Gerät 1 zur quantitativen Bestimmung des in einer vorgegebenen Menge einer Substanz, insbesondere eines Öles, vorhandenen Wassergehalts und/oder dessen Basenzahl durch Zugabe eines mit Wasser bzw. Base ein gasförmiges Produkt bildenden Reagenzes, umfassend ein gasdicht verschließbares, aus mindestens zwei Teilen 2, 3 bestehendes Gehäuse definierten Rauminhaltes, das mit einem Gasdruckmesser 5 verbunden ist, und eine im Gehäuse 1 angeordnete Zuteilvorrichtung 7 für das Reagenz, bei dem der Gasdruckmesser 5 in einem Deckelteil 2 des Gehäuses und die Zuteilvorrichtung als Ausnehmung 7 in der Bodenwand 6 des Gehäuses ausgebildet ist, umfaßt mindestens eine weitere Ausnehmung 8 in der Bodenwand 6 des Gehäuses zur Aufnahme einer weiteren Flüssigkeit. Das Gerät ist einfach zu handhaben, zur Durchführung der Bestimmung sind nur wenige Einzelteile erforderlich; das verwendete Reagenz ist nicht als Gefahrstoff klassifiziert, und das Gerät kann wahlweise zur Wassergehalts- wie auch zur Basenzahlbestimmung eingesetzt werden.

## Beschreibung

Die Erfindung betrifft ein Gerät sowie ein Verfahren zur quantitativen Bestimmung des in einer vorgegebenen Menge einer Substanz, insbesondere eines Öls, vorhandenen Wassergehalts und/oder dessen Basenzahl durch Zugabe eines mit Wasser bzw. Base ein gasförmiges Produkt bildenden Reagenzes, umfassend ein gasdicht verschließbares, aus mindestens zwei Teilen bestehendes Gehäuse definierten Rauminhaltes, das mit einem Gasdruckmesser verbunden ist, und eine innerhalb des Gerätes angeordnete Zuteilvorrichtung für das Reagenz. Ferner betrifft die Erfindung ein Reagenz für die quantitative Bestimmung des in einer vorgegebenen Menge einer Substanz, insbesondere eines Öls, vorhandenen Wassergehalts.

Die quantitative Bestimmung des Wassergehaltes sowie der Basenzahl hat zahlreiche Anwendungsbereiche. Entsprechend gibt es zu diesem Zweck verschiedene Geräte und Verfahren, von denen ein großer Teil jedoch auf den Laboreinsatz zugeschnitten ist. Allerdings gibt es viele Bereiche, in denen ein solches Gerät bzw. ein solches Verfahren nicht zum Einsatz kommen kann, da es zu aufwendig bzw. kompliziert ist, das Gerät nicht für den mobilen Einsatz geeignet ist oder die Meßbedingungen nicht eingehalten werden können.

Ein wichtiger Einsatzbereich für die Bestimmung des Wassergehaltes sowie der Basenzahl ist die Verwendung für die Überprüfung von Schmierölen, Hydraulikölen oder Brennstoffen an Bord von Schiffen. Ein weiterer wichtiger Anwendungsbereich ist der Einsatz für die Vorortanalyse in der Industrie für den Servicebereich bzw. für Betreiber von Anlagen. Die Öle/Brennstoffe dürfen einen bestimmten Wassergehalt nicht überschreiten, um keine Schädigung des Motors hervorzurufen. Bevor die entsprechenden Stoffe eingesetzt werden können, muß sichergestellt werden, daß sie einen bestimmten Wassergehalt nicht überschreiten. Eine komplizierte Laborvorrichtung ist an Bord von Schiffen nicht handhabbar. Es ist daher erforderlich, über ein Meßgerät zu verfügen, das handliche Abmessungen aufweist und einfach zu bedienen ist.

Zu diesem Zweck schlägt die DE 25 19 895 ein Gerät, umfassend ein gasdicht verschließbares Gehäuse definierten Rauminhaltes, das mit einem Gasdruckmesser verbunden ist, und eine im Gerät angeordnete Zuteilvorrichtung für das Reagenz, vor, bei dem die Zuteilvorrichtung als eine auf dem Flüssigkeitsgemisch schwimmende Schale ausgebildet ist, die eine bestimmte Menge des Reagenzes aufnehmen und bei leichtem Schwenken des Gerätes an das Flüssigkeitsgemisch abgeben kann. Dabei besteht das Gerät aus einem Gehäuse, das aus einem Unterteil und einem mit diesem verschraubbaren Oberteil zusammengesetzt ist. Entsprechende Geräte sind seit vielen Jahren so und auch in abgewandelter Form auf dem Markt. In einer Abwandlung ist beispielsweise im oberen Bereich des Gehäuseunterteils eine Rinne vorgesehen, in die das Reagenz einzufüllen ist, während die zu untersuchende Substanz nebst Lösungsmittel in das Gehäuseunterteil gefüllt wird.

Diese Geräte weisen jedoch einige Nachteile auf, die die Anwendung in der Praxis erschweren. Für die Bestimmung des Wassergehaltes ist es sehr wichtig, daß das Reagenz erst dann mit dem zu bestimmenden Flüssigkeitsgemisch in Berührung kommt, wenn Ober- und Unterteil des Gehäuses dicht miteinander verbunden sind, damit nicht frühzeitig eine Reaktion eintritt und kein sich entwickelndes Gas entweichen kann, da dies das Meßergebnis verfälschen würde. Wenn man gemäß der DE 25 19 895 zunächst das zu bestimmende Gemisch sowie ein Lösungsmittel in das Gehäuse einfüllt, ist es nicht ganz einfach, die schwimmende Schale, die das Reagenz enthält, auf dem mit Lösungsmittel versetzten Gemisch zu plazieren, ohne daß etwas von dem Reagenz ausläuft. Die Schale muß mit einer Pinzette eingesetzt werden, was große Vorsicht und Genauigkeit erfordert. Bei hoher See wird das Arbeiten zusätzlich behindert. Des weiteren kommt es bei der Wassergehaltsbestimmung darauf an, daß das Volumen der einzufüllenden Reagenzien sehr exakt abgemessen ist. Fehler im Volumen der Reagenzien wirken sich unmittelbar auf das vorhandene Gasvolumen aus und verfälschen das Meßergebnis. Da das Verhältnis von Gasvolumen zu Flüssigkeitsvolumen etwa 1:5 ist, ist die Fehlerquelle recht groß. Außerdem birgt die Verwendung von zwei unterschiedlichen Flüssigkeiten, dem Reagenz sowie einem zusätzlichen Lösungsmittel, weitere Fehlerquellen.

Bei der oben ebenfalls beschriebenen Abwandlung entfällt zwar das Hantieren mit einer Pinzette, in der Praxis ist die Lösung jedoch vollkommen unbrauchbar, da es für eine Vermischung von Reagenz und zu untersuchender Substanz erforderlich ist, das Gerät auf den Kopf zu stellen. Da sich im Gehäuseoberteil jedoch das Ventil des Druckmeßgerätes befindet, läuft durch das Umdrehen des Gefäßes Flüssigkeit in das Ventil und damit in das Druckmeßgerät, so daß dieses nach kurzer Zeit unbenutzbar wird, insbesondere angesichts der Tatsache, daß es sich bei den verwendeten Substanzen um aggressive Stoffe handelt, die besonders schnell zu einer Beeinträchtigung des Meßgerätes führen.

Des weiteren hat sich die Verwendung eines Schraubverschlusses als nachteilig herausgestellt. Der Verschluß soll möglichst dicht sein, um kein sich bei der Reaktion entwickelndes Gas entweichen zu lassen. Es hat sich jedoch gezeigt, daß bei derart dichten Schraubverschlüssen beim Verschließen bereits ein Vordruck im Behälter entsteht, der das Meßergebnis verfälschen kann.

Außerdem wird bei allen bisher verwendeten Meßverfahren gemäß DE 25 19 895 sowie auch in den abgewandelten Formen mindestens eine Chemikalie verwendet, die hochreaktiv und deshalb als Gefahrstoff klassifiziert ist. Daraus ergeben sich erhebliche Transportprobleme, da diese Gefahrstoffe nur mit speziellen Frachtflugzeugen weltweit verschickt werden können. Auch von Fähren, normalen Straßen- und Schienenfahrzeugen dürfen diese Gefahrstoffe nicht transportiert werden. Daraus ergibt sich das Problem, daß die Reagenzien nicht überall dorthin transportiert werden können, wo sie auch benötigt werden. Da es sich bei den Reagenzien um Verbrauchsmaterialien handelt, ergeben sich erhebliche Schwierigkeiten bei der Nachlieferung. Außerdem übersteigen die Transportkosten häufig den Warenwert um ein weites.

Darüber hinaus sind bei dem bisherigen Meßverfahren zwei unterschiedliche Meßgeräte für die Bestimmung von Wassergehalt und Basenzahl erforderlich. Zwar sind die Meßabläufe ähnlich, aber es sind unterschiedliche Reagenzien einzufüllen, was eine Verwechslung ermöglicht und damit eine große Fehlerquelle darstellt. Schließlich sind zur Bestimmung des Wassergehaltes mit dem Gerät gemäß der DE 25 19 895 bzw. deren abgewandelter Formen zahlreiche Einzelteile erforderlich, was die Handhabung erschwert.

Eine Aufgabe der vorliegenden Erfindung ist es daher, ein Gerät zur quantitativen Bestimmung des in einer vorgegebenen Menge einer Substanz, insbesondere eines Öls, vorhandenen Wassergehalts und/oder dessen Basenzahl durch Zugabe eines mit Wasser bzw. Base ein gasförmiges Produkt bildenden Reagenzes zu schaffen, das diese Nachteile beseitigt, das insbesondere die Zahl der erforderlichen Einzelteile reduziert, die Handhabbarkeit vereinfacht sowie eine genauere Messung ermöglicht.

Diese Aufgabe wird erfindungsgemäß durch ein Gerät der eingangs genannten Art gelöst, bei dem der Gasdruckmesser in einem Deckelteil des Gehäuses und die Zuteilvorrichtung als Ausnehmung in der Bodenwand des Gehäuses ausgebildet ist und das Gerät mindestens eine weitere Ausnehmung in der Bodenwand des Gehäuses zur Aufnahme einer weiteren Flüssigkeit umfaßt.. Durch diese Ausbildung wird sichergestellt, daß zu untersuchende Substanz und Reagenz nicht frühzeitig unerwünscht miteinander in Kontakt geraten. Dadurch daß sowohl die Zuteilvorrichtung als auch die weitere Ausnehmung zur Aufnahme der zu untersuchenden Substanz als Ausnehmungen im Gehäuse ausgebildet sind, ist die Handhabung gegenüber dem Stand der Technik erheblich vereinfacht, da es nicht erforderlich ist, ein kleines Schälchen, beispielsweise mit Hilfe einer Pinzette, auf eine Flüssigkeit im Meßgerät aufzusetzen, was großes Geschick und vorsichtige Handhabung erforderlich macht, wenn man vermeiden will, daß das Reagenz zu früh mit der zu untersuchenden Substanz in Kontakt gerät. Auch ist ein Vermischen durch einfaches Umschwenken des Gerätes möglich. Ein auf-den-Kopf-Stellen, das zwangsläufig mit einer Beschädigung der Druckmeßvorrichtung im System verbunden ist, ist hingegen nicht erforderlich.

In einer vorteilhaften Ausgestaltungsform weist das Gerät Mittel zur Befüllung mit vorgegebenem Volumen auf, die in Form von Einheitsstrichen oder als Ausnehmung mit definiertem Volumen ausgebildet sein können. Auf diese Weise entfällt zumindest ein weiteres Teil zur Durchführung der Messung, wenn man die Ausnehmung zur Aufnahme des Reagenzes entsprechend ausbildet, denn es ist kein Meßbecher o.ä. für die Abmessung des Reagenzes erforderlich. Die Wassergehaltsbestimmung kann somit mit denkbar geringem Geräteaufwand durchgeführt werden, was die Handhabung vereinfacht und den Reinigungsaufwand sowie eine evt. Entsorgung minimiert.

Des weiteren ist es von Vorteil, wenn die Gehäuseteile durch einen Bajonettverschluß miteinander verbunden sind. Ein Bajonettverschluß ermöglicht ein einfaches gasdichtes Verschließen des Gehäuses. Es wird jedoch vermieden, daß - wie beim Schraubverschluß zu beobachten - bereits durch das Verschließen ein Vordruck aufgebaut wird, der das Meßergebnis verfälschen kann. Die Verwendung eines Bajonettverschlusses erhöht somit die Meßgenauigkeit des Gerätes.

Besonders geeignet ist das Gerät, wenn es aus einem Kunststoffmaterial hergestellt ist, das einen im Vergleich zu dem herkömmlich verwendeten Aluminium eine geringere Wärmedurchgangszahl aufweist. Durch diese Materialwahl kann die Meßgenauigkeit weiter erhöht werden, da Ungenauigkeiten, die durch die Abhängigkeit des Drucks von der Temperatur andernfalls auftreten könnten, vermieden werden.

Welche Art Gasdruckmesser verwendet wird, hängt von der zu untersuchenden Substanz ab. Das Gerät ist, wie oben erwähnt, sowohl geeignet, um den Wassergehalt in Ölen und Brennstoffen oder beispielsweise in feuchten Sänden zu ermitteln, wie auch, um die Basenzahl (BN = base number) von Schmierölen zu ermitteln. Das Gerät eignet sich für beide Arten der Bestimmung, Wassergehalt und Basenzahl; die Durchführung der Tests unterscheidet sich lediglich durch die Art des verwendeten Reaganzes. Die Menge des zu verwendenden Reagenz sowie die Menge des zu messenden Substanz bleibt aber bei beiden Messungen gleich. Das zu verwendenden Druckmeßgerät wird in Abhängigkeit des zu erwartenden Wasser- bzw. Basengehalts gewählt. So sind die Geräte für die normale Wassergehaltsbestimmung in Ölen und Brennstoffen auf bis zu 1 bar ausgelegt. Es ist jedoch auch möglich, sehr viel empfindlichere Manometer einzusetzen, wenn beispielsweise im Bereich von Treibstoffen für Helikopter oder Kampfflugzeuge, bei denen der Wassergehalt deutlich unter den Werten für andere Treibstoffe liegt, der Wassergehalt bestimmt werden soll. Des gleichen können sowohl mechanische wie auch elektronische Druckmeßinstrumente zum Einsatz kommen. Bei mechanischen Druckmeßinstrumenten ist vorteilhafterweise eine doppelte Skalierung, zum einen für die Wassergehaltsbestimmung, zum anderen für die Bestimmung der Basenzahl vorgesehen. Bei elektronischen Druckmeßinstrumenten kann ein Schalter vorgesehen sein, durch den die gewünschte Bestimmungsart ausgewählt wird.

Gemäß einem weiteren Aspekt der vorliegenden Erfindung wird ein Verfahren zur quantitativen Bestimmung des in einer vorgegebenen Menge einer Substanz, insbesondere eines Öls, vorhandenen Wassergehalts und/oder dessen Basenzahl durch Zugabe eines mit Wasser bzw. Base ein gasförmiges Produkt bildenden Reagenzes vorgeschlagen. Das bisher angewandte Verfahren, wie es in der DE 25 19 895 teilweise beschrieben ist, weist in der Praxis einige Nachteile auf.

Entsprechend dem bisherigen Verfahren wurde zunächst eine Probe der zu untersuchenden Substanz, z.B. eines Schmieröls, entnommen. 5 ml dieses Öls wurden mit einer Spritze abgemessen und in das in der DE 25 19 895 beschriebene Meßgerät gefüllt. Dazu wurden 15 ml einer Flüssigkeit gegeben, die als Lösungsmittel und Dispersionsmittel wirkte. Zur Abmessung wurde ein Meßzylinder verwendet. Von dem eigentlichen Reagenz wurde eine vorgegebene Zahl Tropfen auf ein Schälchen gegeben, das dann vorsichtig mit einer Pinzette auf das in dem Meßgerät vorhandene Flüssigkeitsgemisch plaziert wurde. Das Gerät wurde verschlossen und geschüttelt, um die Substanzen zu vermischen und die Reaktion auszulösen.

Dieses Verfahren ist insbesondere in zweierlei Hinsicht nachteilig. Zum einen ist das Reagenz hochreaktiv und als Gefahrstoff klassifiziert. Da das Reagenz ein Verbrauchsmaterial darstellt, das an allen Orten der Welt kurzfristig verfügbar sein sollte, wirft diese Klassifizierung Probleme auf, da das Reagenz nicht mit jedem beliebigen Flugzeug an den Bedarfsort gebracht werden kann, sondern nur mit speziellen Frachtflugzeugen, die jedoch bei weitem nicht jeden Flughafen anfliegen. Vielmehr ist eine langwierige Verschiffung erforderlich. Zum anderen ist das Verfahren, wie auch oben schon dargelegt, verhältnismäßig umständlich. Es ist ein Hantieren mit zahlreichen Einzelteilen notwendig. Vor allem das Einsetzen des kleinen Schälchens mit Reagenz in das Flüssigkeitsgemisch erfordert großes Geschick und vorsichtiges Arbeiten, da im Falle des vorzeitigen in-Kontakt-Tretens von Reagenz und zu untersuchender Substanz das Meßergebnis verfälscht wird und der Versuch entsprechend wiederholt werden muß.

Eine weitere Aufgabe der Erfindung ist es daher, ein Verfahren zur Verfügung zu stellen, das die genannten Nachteile beseitigt und insbesondere auf einfache Weise von beliebigen Personen ohne große Schulung oder Einweisung durchgeführt werden kann.

Diese Aufgabe wird durch ein Verfahren zur quantitativen Bestimmung des in einer vorgegebenen Menge einer Substanz, insbesondere eines Öls, vorhandenen Wassergehalts und/oder dessen Basenzahl durch Zugabe eines mit Wasser bzw. Base ein gasförmiges Produkt bildenden Reagenzes in einem gasdicht verschließbaren Gerät, insbesondere unter Verwendung des erfindungsgemäßen Gerätes gelöst, das die folgenden Schritte umfaßt:
- Einfüllen der zu bestimmenden Substanz in eine Ausnehmung in der Bodenwand des Gerätes;
- Befüllen einer zweiten Ausnehmung in der Bodenwand des Gerätes in räumlicher Trennung von der Substanz mit einem einzigen aus dem mit dem Wasser bzw. der Base reagierenden Reagenz;
- Verschließen des Geräts und anschließendes Schütteln desselben;
- Ablesen der sich entwickelnde Gasmenge über ein Meßgerät.

Durch dieses Verfahren unter Verwendung eines einzigen Reagenzes, das die zusätzliche Verwendung eines Lösungsmittels überflüssig macht, ist zum die Handhabung erheblich vereinfacht, da zum einen zusätzlich zur Probe statt bisher zwei nur noch einen Lösung eingefüllt werden muß. Des weiteren ist kein umständliches Einsetzen von zusätzlichen Teilen in das Gerät erforderlich; auch tritt keine Beschädigung des Druckmeßgerätes durch eintretende Flüssigkeit ein, da kein auf-den-Kopf-Stellen des Gerätes erforderlich ist. Vielmehr ist ein Schütteln durch im wesentlichen horizontale Bewegung bzw. ein kräftiges Umschwenken ausreichend.

Gemäß einem weiteren Aspekt der vorliegenden Erfindung wird ein Reagenz zur quantitativen Bestimmung des in einer vorgegebenen Menge einer Substanz, insbesondere eines Öls, vorhandenen Wassergehalts zur Verfügung gestellt, das eine Lösung aus wasserfreiem rein mineralischem Grundöl (Transformatorenöl), enthaltend weniger als 5% Calciumhydrid (CaH₂), vorzugsweise ungefähr 1% Calciumhydrid ist.

Durch das erfindungsgemäße Reagenz wird die Handhabbarkeit wie auch die Anwendbarkeit der quantitativen Wassergehaltsbestimmung sehr stark verbessert, denn das Reagenz ist nicht als Gefahrstoff klassifiziert und kann damit ohne Schwierigkeiten auch beispielsweise per Flugzeug transportiert werden, was die kurzfristige Verfügbarkeit an beliebigen Orten der Welt stark erhöht. Auch die Aufbewahrung des Reagenzes sowie die Handhabung ist weniger kritisch und somit deutlich vereinfacht. Ferner entfällt bei der Wassergehaltbestimmung der Zusatz eines weiteren Verdünnungsmittels zur zu messenden Substanz. Es ist also lediglich erforderlich, zum einen die zu bestimmende Probe abzumessen, zum anderen das Reagenz, 1%ige Calciumhydridlösung in Transformatorenöl, einzufüllen. Bei Verwendung des erfindungsgemäßen Gerätes geschieht das Abmessen des Reagenzes dabei einfach dadurch, daß die Ausnehmung, in die das Reagenz gefüllt wird, ein definiertes Volumen bzw. einen Eichstrich aufweist.

Als Reagenz sind auch andere Substanzen denkbar, die mit der zu bestimmenden Substanz unter Bildung eines gasförmigen Stoffes reagieren.

Die Erfindung stellt somit ein Analysesystem zur quantitativen Bestimmung des in einer vorgegebenen Menge eines Gemisches vorhandenen Wassergehalts und/oder dessen Basenzahl durch Zugabe eines mit Wasser bzw. Base ein gasförmiges Produkt bildenden Reagenzes zur Verfügung, das aus einem vorstehend beschriebenen Gerät, einem einzigen vorgefertigten Reagenz sowie einem Mittel zum Abmessen der zu bestimmenden Substanz, vorzugsweise einer Spritze, besteht. Dieses erfindungsgemäße Analysesystem ist gegenüber herkömmlich bekannten Systemen stark vereinfacht. Das bisherige System bestand aus etwa acht Einzelteilen; das neue System kommt mit drei Teilen (Reaktionsgerät, Reagenz, Mittel zum Abmessen der zu bestimmenden Substanz, z.B. einer Spritze) aus.

Das Gesamtsystem wird somit kompakter. Es ist weniger Verbrauchsmaterial erforderlich, und die Handhabbarkeit ist stark vereinfacht, insbesondere da das Einsetzen eines Schälchens mit hoch reaktivem Reagenz auf die zu untersuchende Substanz entfällt. Außerdem sind wesentliche Fehlerquellen auf ein Minimum reduziert und die Versorgung mit Verbrauchsmaterialien ist wesentlich vereinfacht worden. Des weiteren ist zur Messung der beiden unterschiedlichen Größen (Wassergehalt und BN) nur noch ein Meßgerät erforderlich.

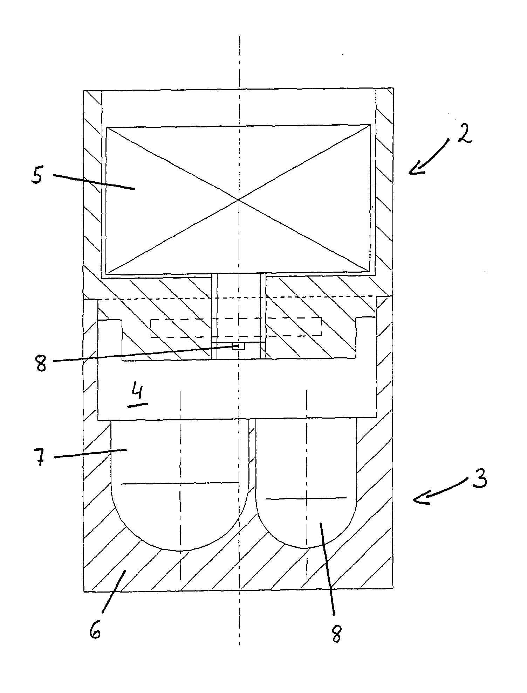

Weitere Vorteile und Anwendungsmöglichkeiten gehen aus dem Ausführungsbeispiel hervor, das anhand der beigefügten Zeichnungen im folgenden näher beschrieben werden soll. Dabei zeigt die Figur:
einen Längsschnitt eines erfindungsgemäßen Gerätes zur quantitativen Bestimmung des in einer vorgegebenen Menge einer Substanz, insbesondere eines Öles, vorhandenen Wassergehalts und/oder dessen Basenzahl durch Zugabe eines mit Wasser bzw. Base ein gasförmiges Produkt bildenden Reagenzes.

Das in der Figur gezeigte Gerät 1 umfaßt ein aus mindestens zwei Teilen 2, 3 bestehendes Gehäuse 2, 3 sowie einen Innenraum 4. Im Oberteil 2 des Gehäuses befindet sich ein Druckmesser 5, der über einen Verbindungskanal sowie ein Ventil 8 mit dem Innenraum 4, der ein bekanntes Volumen aufweist, verbunden ist. Das Gehäuseoberteil 2 ist mit dem Gehäuseunterteil 3 mittels eines Bajonettverschlusses verschließbar. Zu Dichtungszwecken ist ein O-Ring vorgesehen. In der Bodenwand 6 des Gehäuseunterteils 3 sind zwei Ausnehmungen 7, 8 vorgesehen, von denen mindestens eine ein definiertes Volumen aufweist. Wahlweise kann auch eine Markierung vorgesehen sein, die angibt, bis wohin die Substanz einzufüllen ist.

Zur Bestimmung des Wassergehaltes einer Ölprobe wird eine der beiden Ausnehmungen 7 bis zur Markierung mit dem Reagenz, bestehend aus einer 1%igen Calciumhydridlösung in wasserfreiem rein mineralischen Grundöl, gefüllt. Mit Hilfe einer Spritze wird eine definierte Menge des zu analysierenden Öls entnommen. Diese Probe wird in die zweite Ausnehmung 8 im Gehäuseboden gefüllt. Das Gerät wird durch Aufsetzen des Gehäuseoberteils 2 durch den Bajonettverschluß verschlossen. Durch das einfache, schnelle Verschließen, das der Bajonettverschluß gewährleistet, wird vermieden, daß sich im Gerät ein Vordruck aufbaut, der das Meßergebnis verfälschen könnte. Das Gerät wird nun ca. 10 Sekunden kräftig umgeschwenkt bzw. durch Bewegung in im wesentlichen horizontaler Richtung geschüttelt. Nach jeweils einer kurzen Pause wird das Schwenken alle 1-2 Minuten solange wiederholt, bis die Anzeige auf dem Druckmesser konstant ist. Das in der Probe vorhandene Wasser hat mit dem Reagenz unter Wasserstoffbildung reagiert. Die Menge des gebildeten Wasserstoffs kann anhand des Druckes bestimmt werden.

Der Druckmesser kann eine auf den Wassergehalt und/oder die Basenzahl geeichte Skala aufweisen. Soll das Gerät sowohl zur Wassergehalts- als auch zur Bestimmung der Basenzahl eines Schmieröls eingesetzt werden, ist es ebenfalls möglich, eine Skala, die den Druck anzeigt, zu wählen und dem Benutzer eine Eichkurve mitzugeben, anhand derer er den abgelesenen Druck in einen Wasseranteil bzw. eine Basenzahl umrechnen kann.

## Patentansprüche

1. Gerät (1) zur quantitativen Bestimmung des in einer vorgegebenen Menge einer Substanz, insbesondere eines Öles, vorhandenen Wassergehalts und/oder dessen Basenzahl durch Zugabe eines mit Wasser bzw. Base ein gasförmiges Produkt bildenden Reagenzes, umfassend ein gasdicht verschließbares, aus mindestens zwei Teilen (2, 3) bestehendes Gehäuse definierten Rauminhaltes, das mit einem Gasdruckmesser (5) verbunden ist, und eine im Gehäuse angeordnete Zuteilvorrichtung (7) für das Reagenz, **dadurch gekennzeichnet, daß** der Gasdruckmesser (5) in einem Deckelteil (2) des Gehäuses und die Zuteilvorrichtung als Ausnehmung (7) in der Bodenwand (6) des Gehäuses ausgebildet ist und das Gerät (1) mindestens eine weitere Ausnehmung (8) in der Bodenwand (6) des Gehäuses zur Aufnahme einer weiteren Flüssigkeit umfaßt.

2. Gerät (1) nach Anspruch 1, **dadurch gekennzeichnet, daß** das Gerät (1) Mittel zur Befüllung mit vorgegebenem Volumen aufweist.

3. Gerät (1) nach Anspruch 2, **dadurch gekennzeichnet, daß** das Mittel zur Befüllung mit vorgegebenem Volumen als Einheitsstriche ausgebildet ist.

4. Gerät (1) nach Anspruch 2, **dadurch gekennzeichnet, daß** das Mittel zur Befüllung mit vorgegebenem Volumen als Ausnehmung (7, 8) definierten Volumens ausgebildet ist.

5. Gerät (1) nach einem der Ansprüche 1 bis 4, **dadurch gekennzeichnet, daß** die Gehäuseteile (2, 3) durch einen Bajonettverschluß verschließbar sind.

6. Gerät (1) nach einem der Ansprüche 1 bis 5, **dadurch gekennzeichnet, daß** das Gehäuse aus einem Kunststoffmaterial besteht.

7. Verfahren zur quantitativen Bestimmung des in einer vorgegebenen Menge einer Substanz, insbesondere eines Öls, vorhandenen Wassergehalts und/oder dessen Basenzahl durch Zugabe eines mit Wasser bzw. Base ein gasförmiges Produkt bildenden Reagenzes in einem gasdicht verschließbaren Gerät, insbesondere unter Verwendung des Gerätes nach einem der Ansprüche 1 bis 6, **gekennzeichnet durch** die folgenden Schritte:
- Einfüllen der zu bestimmenden Substanz in eine Ausnehmung in der Bodenwand des Gerätes;
- Befüllen einer zweiten Ausnehmung in der Bodenwand des Gerätes in räumlicher Trennung von der Substanz mit einem einzigen aus dem mit dem Wasser bzw. der Base reagierenden Reagenz;
- Verschließen des Geräts und anschließendes Schütteln desselben;
- Ablesen der sich entwickelnde Gasmenge über ein Meßgerät.

8. Reagenz zur quantitativen Bestimmung des in einer vorgegebenen Menge einer Substanz, insbesondere eines Öls, vorhandenen Wassergehalts, **dadurch gekennzeichnet, daß** das Reagenz eine Lösung aus wasserfreiem rein mineralischem Grundöl, enthaltend weniger als 5% Calciumhydrid, ist.

9. Reagenz nach Anspruch 8, **dadurch gekennzeichnet, daß** es ungefähr 1% Calciumhydrid enthält.

10. Analysesystem zur quantitativen Bestimmung des in einer vorgegebenen Menge eines Gemisches vorhandenen Wassergehalts und/oder dessen Basenzahl durch Zugabe eines mit Wasser bzw. Base ein gasförmiges Produkt bildenden Reagenzes, bestehend aus einem Gerät nach einem der Ansprüche 1 bis 6, einem einzigen vorgefertigten Reagenz, insbesondere einem Reagenz gemäß Anspruch 8 oder 9, sowie einem Mittel zum Abmessen der zu bestimmenden Substanz, vorzugsweise einer Spritze.
